# NEUE EUROPÄISCHE PATENTSCHRIFT

(11) **EP 1 206 488 B2**
(45) Veröffentlichungstag und Bekanntmachung des Hinweises auf die Entscheidung über den Einspruch: **14.10.2015**
(45) Hinweis auf die Patenterteilung: 08.06.2005
(21) Anmeldenummer: 00958467.3
(22) Anmeldetag: 18.08.2000
(51) Int. Cl.: C07K 14/415, A61K 39/36, G01N 33/68

(54) **VERFAHREN ZUR ISOLIERUNG UND AUFREINIGUNG VON GRÄSERPOLLENALLERGENEN**
METHOD FOR ISOLATING AND PURIFYING GRASS POLLEN ALLERGENS
PROCEDE D'ISOLATION ET DE PURIFICATION D'ALLERGENES DE POLLENS DE PLANTES HERBACEES

(30) Priorität: 24.08.1999 DE 19939982
(43) Veröffentlichungstag der Anmeldung: 22.05.2002
(73) Patentinhaber: Merck Patent GmbH, 64293 Darmstadt (DE)
(72) Erfinder: SUCK, Roland, D-22303 Hamburg (DE); CROMWELL, Oliver, D-21465 Wentorft (DE); FIEBIG, Helmut, D-21493 Schwarzenbek (DE)
(86) Internationale Anmeldenummer: PCT/EP2000/008059
(87) Internationale Veröffentlichungsnummer: WO 2001/013946

(56) Entgegenhaltungen:
- B FAHLBUSCH ET AL.: "Application of reversed-phase high-performance liquid chromatography in the purification of major allergens from grass pollen " JOURNAL OF IMMUNOLOGICAL METHODS, Bd. 194, Nr. 1, 1996, Seiten 27-34, XP002161720 NEW YORK US in der Anmeldung erwähnt
- DATABASE CHEMABS [Online] CHEMICAL ABSTRACTS SERVICE, COLUMBUS, OHIO, US; BOLZACCHINI, EZIO ET AL: "Purification of Phleum pratense pollen extract by immunoaffinity chromatography and high-performance ion-exchange chromatography" retrieved from STN Database accession no. 115:112253 CA XP002161722 & J. CHROMATOGR. (1991), 548(1-2), 229-34 , 1991,
- R SUCK ET AL.: "Rapid and efficient purification of Phleum pratense major allergens Phl p 1 and group Phl p 2/3 using a two-step procedure" JOURNAL OF IMMUNOLOGICAL METHODS., Bd. 229, November 1999 (1999-11), Seiten 73-80, XP002161721 ELSEVIER SCIENCE PUBLISHERS B.V.,AMSTERDAM., NL ISSN: 0022-1759

## Beschreibung

Die Erfindung betrifft ein Verfahren zur schnellen und effektiven Isolierung und Reinigung von fünf Allergenen der Gruppen 1, 2, 3, 10 und 13 aus Gräserpollen. Als natürlicher Rohstoff für die Allergenreinigung dienen die Pollen von Süßgräsern, wie z.B. von *Phleum pratense*. Die Aufreinigung basiert auf einer erfindungsgemäßen Kombinantion von hydrophober Interaktionschromatographie, Gelfiltration und Kationaustauschchromatographie. Die so erhaltenen Proteine können zur verbesserten Diagnostik von Pollenallergien sowie für pharmazeutische Zubereitungen für die Therapie von pollenallergischen Krankheiten verwendet werden.

Allergien vom Typ 1 haben weltweite Bedeutung. Bis zu 20% der Bevölkerung in industrialisierten Ländern leiden unter Beschwerden wie allergischer Rhinitis, Konjunktivitis oder Bronchialasthma, die durch in der Luft befindliche Allergene (Aeroallergene), die von unterschiedlichen Quellen wie Pflanzen, Milben, Katzen oder Hunden freigesetzt werden, hervorgerufen werden. Bis zu 40% dieser Typ 1-Allergiker wiederum zeigen spezifische IgE-Reaktivität mit Allergenen aus Gräserpollen (Freidhoff et al., 1986, J Allergy Clin Immunol 78, 1190-201).

Bei den Typ 1-Allergie auslösenden Substanzen handelt es sich um Proteine, Glykoproteine oder Polypeptide. Diese Allergene reagieren nach Aufnahme über die Schleimhäute mit den bei sensibilisierten Personen an der Oberfläche von Mastzellen gebundenen IgE-Molekülen. Werden zwei oder mehr IgE-Moleküle durch ein Allergen miteinander vernetzt, führt dies zur Ausschüttung von Mediatoren (z.B. Histamin, Prostaglandinen) und Zytokinen durch die Effektorzelle und damit zu den entsprechenden klinischen Symptomen.

In Abhängigkeit von der relativen Häufigkeit der Allergiker, die IgE-Antikörper gegen bestimmte Allergene aufweisen, wird zwischen Major- und Minorallergenen unterschieden. Im Fall vom Lieschgras (Phleum pratense) sind bislang Phl p 1 (Petersen et al., 1993, J. Allergy Clin. Immunol. 92, 789-796), Phl p 5 (Matthiesen und Löwenstein, 1991, Clin. Exp. Allergy 21, 297-307; Petersen et al., 1992), Phl p 6 (Petersen et al., 1995, Int. Arch. Allergy Immunol. 108, 49-54) und Phl p 2/3 (Dolecek et al., 1993) als Majorallergene und Phl p 4 (Löwenstein, 1978, Prog. Allergy 25, 1-62) sowie Gruppe 10 und 11 aus Lolium perenne (Ansari et. al., 1987, J. Allergy Clin. Immunol. 80, 229-235) als Minorallergene charakterisiert worden. Außerdem ist kürzlich ein weiteres hochmolekulares Majorallergen beschrieben worden, das als Phl p 13 bezeichnet wurde. Die Allergene der Gruppe 1 und 13 sind glykosyliert.

Im Zusammenhang mit der vorliegenden Erfindung sind die Allergengruppen 1, 2, 3, 10 und 13 von besonderer Bedeutung. Etablierte Reinigungsmethoden der natürlichen Allergene basieren auf der Isolierung von jeweils einzelnen Proteinen. Mittels Affinitätschromatographie unter Verwendung von spezifischen Antikörpern sind z.B. bisher Gruppe 1 Allergene aus *Lolium perenne* (Boutin et al., 1996, Int. Arch. Allergy Immunol. 112, 218-225) und *Phleum pratense* (Grobe et al., 1999, Eur. J. Biochem. 263, 33-40) gereinigt worden. Diese Methode ist hinsichtlich ihrer Kapazität begrenzt und wird unter Verwendung extremen pH durchgeführt, so daß der Erhalt der nativen Konformation nicht sichergestellt werden kann. Andere Verfahren basieren auf verschiedenen mehrstufigen Abfolgen chromatographischer Schritte. Dabei werden jeweils individuelle Allergene erhalten, wie z.B. Gruppe 10 (Ansari et al., 1987, J Allergy Clin Immunol 80, 229-235) oder Gruppe 3 (Ansari et al., 1989, Biochemistry 28, 8665-8670). Andere Allergene gehen bei diesen Methoden verloren oder sind nicht rein darzustellen.

Eine Methode zur Reinigung der Hauptallergene der Gruppe 1 und 5 aus Graspollen wird von Fahlbusch et al. (J. Immunol. Meth. 194 (1996) 27-34) beschrieben. Jedoch ist auch diese Methode einsichtlich ihrer Kapazität begrenzt.

DNA-Sequenzdaten liegen u.a. von Phl p 1 (Laffer et al., 1994, J. Allergy Clin. Immunol. 94, 1190-98; Petersen et al., 1995, J. Allergy Clin. Immunol. 95(5). 987-994), Phl p 5 (Vrtala et al., 1993, J. Immunol. 151 (9), 4773-4781), Phl p 6 (Petersen et al., 1995, Int. Arch. Allergy Immunol. 108 (1), 55-59) und Phl p 2 (Dolecek et. al., 1993, FEBS 335 (3), 299-304) vor. Mit Hilfe von cDNA-Sequenzen ist es möglich, rekombinante Allergene herzustellen, die in der Diagnostik und Therapie Verwendung finden können (Scheiner and Kraft, 1995, Allergy 50, 384-391).

Ein klassischer Ansatz zur wirksamen therapeutischen Behandlung von Allergien stellt die Spezifische Immuntherapie oder Hyposensibilisierung dar (Fiebig, 1995, Allergo J. 4 (6), 336-339; Bousquet et al., 1998, J. Allergy Clin Immunol. 102 (4), 558-562). Dabei werden dem Patienten natürliche Allergenextrakte in steigenden Dosen subkutan injiziert. Allerdings besteht bei dieser Methode die Gefahr von allergischen Reaktionen oder sogar eines anaphylaktischen Schocks. Um diese Risiken zu minimieren werden innovative Präparate in Form von Allergoiden eingesetzt. Dabei handelt es sich um chemisch modifizierte Allergenextrakte, die deutlich reduzierte IgE-Reaktivität, jedoch identische T-Zell-Reaktivität im Vergleich zum nicht behandelten Extrakt aufweisen (Fiebig, 1995, Allergo J. 4 (7), 377-382).

Eine weitergehende Therapieoptimierung wäre mit hochgereinigten Allergenen möglich. Definierte Cocktails aus natürlichen Allergenen können die bisherigen Extrakte ablösen, da diese außer den verschiedenen Allergenen eine größere Zahl von immunogenen, aber nicht allergenen Begleitproteinen, die für die spezifische Immuntherapie nicht notwendig sind, enthalten. Die Verwendung von Allergen- Cocktails läßt auch die Bereitung von patientenspezifischen Allergenmischungen entsprechend des Sensibilisierungsspektrums zu. Realistische Perspektiven, die zu einer sicheren Hyposenibilisierung mit hochreinen natürlichen Allergenen führen können, bieten modifizierte Allergene, bei denen IgE-Epitope durch irreversible Modifikation der Sekundär- und Tertiärstruktur zerstört werden, ohne die für die Therapie essentiellen T-Zell Epitope zu beeinträchtigen.

Die Erfindung kann ebenso in der In-vitro- und In-vivo-Diagnostik von allergischen Erkrankungen, speziell der Pollinosis, vorteilhaft angwendet werden. Dazu werden die gereinigten Allergengruppen zur Detektion von IgE-Antikörpern in etablierten Verfahren eingesetzt.

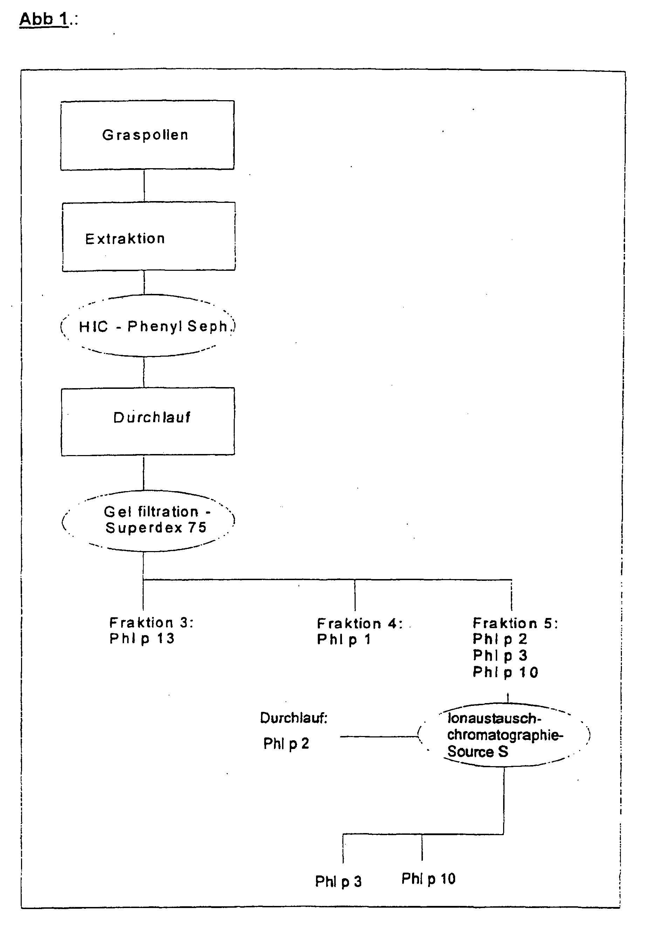

Bei der Erfindung handelt es sich um ein biochemisches Reinigungsverfahren, das über eine effiziente Drei-Stufen-Reinigung zur Isolierung von 4 Majorallergenen und 1 Minorallergen aus wässrigen Kurzzeitpollenextrakten führt. Als natürlicher Rohstoff dienen Pollen der Graminaen, wie z.B. *Phleum pratense, Lolium perenne, Dactylis glomerata, Poa pratensis, Cynodon dactylon, Holcus lanatus* u.a. Abb. 1 gibt das Aufreinigungsschema der 5 genannten Allergene aus Graspollenextrakten wieder. Dabei entsprechen die Allergenbezeichnungen Phl p 1 bis Phl p 13 den ansonsten im Text verwendeten Bezeichnungen Allergenen 1 bis 13.

Gegenstand der Erfindung ist somit ein Verfahren zur Gewinnung von im wesentlich reinen Gras-Allergenen der Gruppen 1, 2, 3, 10, 13, ausgenommen *Phleum pratense*, worin ein wäßriger Extrakt von Pollen der Graminaen hergestellt wird, und die löslichen Bestandteile einer hydrophoben Interaktionchromatographie, einem Gelfiltrations-Schritt und gegebenenfalls einer Kationenaustauscherchromatographie unterzogen werden. Erfindungsgemäß ist auch ein Verfahren zur Gewinnung von im wesentlich reinen Gras-Allergen der Gruppen 1, 2, 3, 10, 13, dadurch gekennzeichnet, daß ein wäßriger Extrakt von Pollen der Graminaen hergestellt wird, und die löslichen Bestandteile einer hydrophoben Interaktionchromatographie einem Gelfiltrations-Schritt und einer Kationenaustauscherchromatographie unterzogen werden.
Erfindungsgemäß können auch mehrere Schritte einer Chromatographie-Art durchgeführt werden, in der Regel ist das Verfahren aber so effektiv, daß jeweils ein Trennungsschritt ausreicht.

Das Verfahren eignet sich in besonderer Weise für die Gewinnung besagter Allergene aus den Pollen der Spezies *Phleum pratense, Lolium perenne, Dactylis glomerata, Festuca pratensis, Holcus lanatus, Poa pratensis, Secale cereale.*

In einer bevorzugten Ausführungsvariante erfolgt die Extraktion mittels Tris/HClgepufferter wäßriger Lösung. Es sind jedoch erfindungsgemäß auch andere bekannte wäßrige Pufferlösungen einsetzbar.

Für die Aufreinigung der genannten Allergene werden die löslichen Bestandteile des Extraktes eingesetzt. Hierzu wird der Extrakt 3 bis 8 Minuten, vorzugsweise 5 Minuten bei 18.000 bis 30.000 x g zentrifugiert und der Überstand zur weiteren Aufreinigung hergenommen. Alternativ kann auch eine Abtrennung der unlöslichen Bestandteile durch andere Methoden, beispielsweise durch Filtration erfolgen.

Der erste chromatographische Aufreinigungsschritt erfolgt mittels hydrophober Interaktions-Chromatographie, beispielsweise an Sepharose®. Hierbei werden viele Verunreinigungen auf dem Träger festgehalten, während die gewünschten Allergene sich im Durchlauf befinden. Entsprechende andere Trägermaterialien können ebenfalls eingesetzt werden.

Gegenstand der Erfindung ist somit ein entsprechendes Verfahren, worin mittels hydrophober Interaktions-Chromatographie die Gras-Allergene der Gruppen 1, 2, 3, 10, 13 von anderen Bestandteilen abgetrennt werden.

Im nachfolgenden Aufreinigungsschritt werden die Gras-Allergene in drei Fraktionen getrennt, wobei die Gruppen 1, 13 jeweils eine Fraktion und die Gruppen 2, 3 und 10 die dritte Fraktion darstellen. Gegenstand der Erfindung ist somit im speziellen ein Verfahren, worin die Allergene der Gruppe 1 und 13 durch einen nachgeschalteten Gelfiltrations-Schritt in separaten Fraktionen erhalten und von den Allergenen der Gruppe 2, 3, und 10 abgetrennt werden.

Letztere können dann erfindungsgemäß durch einen nachfolgenden Chromatographieschritt über einen Kationenaustauscher voneinander getrennt werden. Gegenstand der Erfindung ist daher ein Verfahren, worin die nach dem Gelfiltrations-Schritt erhaltenen Allergene der Gruppe 2, 3 und 10 durch eine nachgeschaltete Kationenaustauschchromatographie voneinander getrennt werden.

Die Identifikation der genannten an sich bekannten Allergene erfolgt entweder über ihre bekannten unterschiedlichen physikalischen, chemischen, biologischen oder immunologischen Eigenschaften, insbesondere mittels isoelektrischer Fokussierung, UV-Absorptionsmessungen, SDS-PAGE und spezifischer Antikörper. Diese Verfahren und Techniken sind bekannt und allgemein beschrieben.

Die Ausbeute der erfindungsgemäß gewonnenen Allergene beträgt 0,5 - 1,5% bezogen auf das ursprünglich eingesetzte Gesamtprotein der Graspollen.

Die Erfindung dient auch zur Verbesserung der In-vivo- und In-vitro-Diagnostik im Rahmen einer Allergen-Komponenten auflösenden Identifizierung des patientenspezifischen Sensibilisierungsspektrums.

Die Erfindung dient ebenfalls zur Herstellung von verbesserten Präparaten zur spezifischen Immuntherapie von Gräserpollenallergien, was durch Abtrennung von immunogenen, aber für die Therapie irrelevanten Extraktbestandteilen erreicht wird. Weiterhin kann durch die chemische Umsetzung der gereinigten Allergene ein Allergoidpräparat erhalten werden.

Im nachfolgenden wird das Verfahren im Detail beschrieben:

Die Reinigung der natürlichen Allergene aus Lieschgraspollen wird in einem Dreischrittverfahren durchgeführt (siehe Abb. 1). Nach der wässrigen Extraktion mit Tris/HCl-gepufferter Lösung (20 mM Tris/HCl, 1 mM EDTA, pH 8.0) von Pollen über 30 Minuten wird der Extrakt durch Zentrifugation, vorzugsweise bei 20.000 x g fünf Minuten lang, separiert. Der Tris/HCl-gepufferte (20 mM Tris/HCl, 1 mM EDTA, pH 8.0) Überstand wird mit 1 M Ammoniumsulfat versetzt und anschließend einer Hydrophoben-Interaktions-Chromatographie (Phenyl-Sepharose High Performance, Pharmacia) unterzogen. Eine typische Säule ist mit 50 bis 100 ml des Trägermaterials gepackt und wird mit einer Flußrate von etwa 5 ml / min betrieben. Die Durchlauffraktion enthält ausschließlich die Proteine von fünf Allergengruppen: Gruppe 1 (30-35 kDa), Gruppe 2 (11 kDa), Gruppe 3 (12 kDa), Gruppe 10 (13 kDa) und Gruppe 13 (55-60 kDa). Es folgt eine Einengung des Volumens vorzugsweise durch Ultrafiltration oder Lyophilisation.

In einem zweiten Schritt werden die Allergene der Gruppen 13 und 1 dann durch Gelfiltration entsprechend ihrer unterschiedlichen Molekularmassen mit Superdex® 75 prep grade (Pharmacia) oder ähnlichen bekannte für diese Zwecke geeignete Trägermaterialien von den niedermolekularen Allergenen getrennt. Das Elutionsmedium ist vorzugsweise 50 mM Ammoniumhydrogencarbonat. Die Säule wird mit einer Flußrate von etwa 5 ml/min betrieben. Man erhält drei Fraktionen, welche die Allergene 1, 13 (jeweils getrennt) und 2, 3,10 (in einer Fraktion) enthalten.

Die niedermolekularen Allergene der Gruppen 2, 3 und 10, welche zusammen in der dritten Fraktion der Gelfiltration eluiert wurden, werden mittels Kationenaustauschchromatographie voneinander getrennt. Hierzu wird die lyophilisierte Probe in einen wäßrigen Puffer, vorzugsweise 20 mM Phosphatpuffer, pH 7.2 aufgenommen und auf eine mit diesem Puffer äquilibrierte Kationenaustauscher-Säule (z.B. Source S ®) aufgetragen. Im Durchlauf findet man das saure Allergen 2. Durch einen Salzgradienten von 0 - 500 mM NaCl über etwa 20 Säulenvolumina werden die gebundenen Allergene 3 und 10 nacheinander eluiert. Damit ist die niedermolekulare Allergengruppe in ihre Einzelallergene separiert. Auch andere Kationenaustauschermaterialien können erfindungsgemäß eingesetzt werden.

Die vorliegende Erfindung ermöglicht also durch das zur Verfügung gestellte erfindungsgemäße Verfahren, welches sich durch die spezielle Abfolge der Chromatographieschritte sowie die Wahl der Chromatographiemedien auszeichnet, eine hochskalierbare, technologisch umsetzbare Produktionsmethode zur Gewinnung von mehreren hochreinen, natürlichen Gräser-Allergenen mit geringem Arbeits-und Zeitaufwand. Da die angewendeten Reinigungsverfahren sehr schonend für Proteine sind, bleiben deren Konformationen und Antigenität erhalten. Dies ist eine Voraussetzung für eine erfolgreiche Diagnostik von allergischen Erkrankungen.

## Patentansprüche

1. Verfahren zur Gewinnung von im wesentlichen reinen Gras-Allergenen der Gruppen 1, 2, 3,10,13, **dadurch gekennzeichnet, dass** ein wässriger Extrakt von Pollen der Graminaen, ausgenommen Phleum pratense, hergestellt wird, und die löslichen Bestandteile einer hydrophoben Interaktionchromatographie, einem Gelfiltrations-Schritt und gegebenenfalls einer Kationenaustauscherchromatographie unterzogen werden.

2. Verfahren gemäß Anspruch 1, **dadurch gekennzeichnet, dass** Pollen der Spezies Lolium perenne, Dactylis glömerata, Festuca pratensis, Holcus lanatus, Poa pratensis, Secale cereale zur Extraktion verwendet werden.

3. Verfahren nach Anspruch 1 oder 2, **dadurch gekennzeichnet, dass** die Extraktion mittels Tris/HCl-gepufferter wässriger Lösung erfolgt.

4. Verfahren nach einem der Ansprüchen 1 bis 3, **dadurch gekennzeichnet, dass** in einem ersten Schritt mittels hydrophober Interaktions-Chromatographie die Gras-Allergene der Gruppen 1, 2, 3, 10, 13 von anderen Bestandteilen abgetrennt werden.

5. Verfahren gemäß Anspruch 4, **dadurch gekennzeichnet, dass** die Allergene der Gruppe 1 und 13 durch einen nachgeschalteten Gelfiltration-Schritt in separaten Fraktionen erhalten und von den Allergenen der Gruppe 2, 3, und 10 abgetrennt werden.

6. Verfahren gemäß Anspruch 5, **dadurch gekennzeichnet, dass** die nach dem Gelfiltrations-Schritt erhaltenen Allergene der Gruppe 2, 3 und 10 durch eine nachgeschaltete Kationenaustauschchromatographie voneinander getrennt werden.

7. Verfahren zur Gewinnung von im wesentlichen reinem Gras-Allergen der Gruppe 13, ausgenommen Phleum pratense, **dadurch gekennzeichnet, dass** ein wässriger Exktrakt von Pollen der Graminaen hergestellt wird und die löslichen Bestandteile einer hydrophoben Interaktionschromatographie und einem Gelfiltrations-Schritt unterzogen werden, wobei bei letzterem Schritt drei Fraktionen erhalten werden, von denen die Gruppe 1 und 13 Allergene jeweils eine Fraktion und die Gruppe 2, 3 und 10 Allergene die dritte Fraktion darstellen.

8. Verfahren zur Gewinnung von im wesentlichen reinen Gras-Allergenen der Gruppen 1, 2, 3,10,13, **dadurch gekennzeichnet, dass** ein wässriger Extrakt von Pollen der Graminaen hergestellt wird, und die löslichen Bestandteile einer hydrophoben Interaktionchromatographie, einem Gelfiltrations-Schritt und einer Kationenaustauscherchromatographie unterzogen werden.

## Claims

1. Method for obtaining essentially pure grass allergens of groups 1, 2, 3, 10, 13, **characterised in that** an aqueous extract of pollen of the Graminae, with the exception of Phleum pratense, is prepared and the soluble constituents are subjected to hydrophobic interaction chromatography, a gel filtration step and optionally cation exchanger chromatography.

2. Method according to Claim 1, **characterised in that** pollen of the species Lolium perenne, Dactylis glomerata, Festuca pratensis, Holcus lanatus, Poa pratensis, Secale cereale are used for the extraction.

3. Method according to Claim 1 or 2, **characterised in that** the extraction is carried out by means of Tris/HCl-buffered aqueous solution.

4. Method according to one of Claims 1 to 3, **characterised in that**, in a first step, the grass allergens of groups 1, 2, 3, 10, 13 are separated off from other constituents by means of hydrophobic interaction chromatography.

5. Method according to Claim 4, **characterised in that** the allergens of groups 1 and 13 are obtained in separate fractions by a subsequent gel filtration step and are separated off from the allergens of groups 2, 3 and 10.

6. Method according to Claim 5, **characterised in that** the allergens of groups 2, 3 and 10 obtained after the gel filtration step are separated from one another by subsequent cation exchange chromatography.

7. Method for obtaining essentially pure grass allergen of group 13, with the exception of Phleum pratense, **characterised in that** an aqueous extract of pollen of the Graminae is prepared, and the soluble constituents are subjected to hydrophobic interaction chromatography and a gel filtration step, the latter step giving three fractions, of which the group 1 and 13 allergens each represent one fraction and the group 2, 3 and 10 allergens represent the third fraction.

8. Method for obtaining essentially pure grass allergens of groups 1, 2, 3, 10, 13, **characterised in that** an aqueous extract of pollen of the Graminae is prepared, and the soluble constituents are subjected to hydrophobic interaction chromatography, a gel filtration step and cation exchanger chromatography.

## Revendications

1. Procédé pour obtenir des allergènes d'herbe des groupes 1, 2, 3, 10, 13 essentiellement purs, **caractérisé en ce qu'**un extrait aqueux de pollen de Graminae, à l'exception de Phleum pratense, est préparé et les constituants solubles sont soumis à une chromatographie par interaction hydrophobe, à une étape de filtration sur gel et en option, à une chromatographie échangeuse de cations.

2. Procédé selon la revendication 1, **caractérisé en ce que** des pollens des espèces Lolium perenne, Dactylis glomerata, Festuca pratensis, Holcus lanatus, Poa pratensis, Secale cereale sont utilisés pour l'extraction.

3. Procédé selon la revendication 1 ou 2, **caractérisé en ce que** l'extraction est mise en oeuvre au moyen d'une solution aqueuse tamponnée Tris/HCl.

4. Procédé selon l'une des revendications 1 à 3, **caractérisé en ce que**, au niveau d'une première étape, les allergènes d'herbe des groupes 1, 2, 3, 10, 13 sont séparés à partir d'autres constituants au moyen d'une chromatographie par interaction hydrophobe.

5. Procédé selon la revendication 4, **caractérisé en ce que** les allergènes des groupes 1 et 13 sont obtenus selon des fractions séparées au moyen d'une étape de filtration sur gel qui suit et sont séparés à partir des allergènes des groupes 2, 3 et 10.

6. Procédé selon la revendication 5, **caractérisé en ce que** les allergènes des groupes 2, 3 et 10 qui sont obtenus après l'étape de filtration sur gel sont séparés les uns des autres au moyen d'une chromatographie échangeuse de cations qui suit.

7. Procédé pour obtenir un allergène d'herbe du groupe 13 essentiellement pur, à l'exception de Phleum pratense, **caractérisé en ce qu'**un extrait aqueux de pollen de Graminae est préparé et les constituants solubles sont soumis à une chromatographie par interaction hydrophobe et à une étape de filtration sur gel, cette dernière étape donnant trois fractions, les allergènes des groupes 1 et 13 représentant chacun une fraction et les allergènes des groupes 2, 3 et 10 représentant la troisième fraction.

8. Procédé pour obtenir des allergènes d'herbe des groupes 1, 2, 3, 10, 13 essentiellement purs, **caractérisé en ce qu'**un extrait aqueux de pollen de la Graminae est préparé et les constituants solubles sont soumis à une chromatographie par interaction hydrophobe, à une étape de filtration sur gel et à une chromatographie échangeuse de cations.
